# EUROPÄISCHE PATENTSCHRIFT

(11) **EP 0 582 248 B1**
(45) Veröffentlichungstag und Bekanntmachung des Hinweises auf die Patenterteilung: **13.03.1996**
(21) Anmeldenummer: 93112349.1
(22) Anmeldetag: 02.08.1993
(51) Int. Cl.: C07C 303/22, C07C 303/32, C07C 309/58

(54) **Verfahren zur Herstellung von 3-Sulfobenzoesäure und deren Alkalisalzen**
Method for the preparation of 3-sulphobenzoic acid and its alkali metal salts
Procédé pour la préparation d'acide 3-sulfobenzoique et ses sels de métaux alcalins

(30) Priorität: 07.08.1992 DE 4226131
(43) Veröffentlichungstag der Anmeldung: 09.02.1994
(73) Patentinhaber: HOECHST AKTIENGESELLSCHAFT, D-65926 Frankfurt am Main (DE)
(72) Erfinder: Meier, Michael, Dr., D-60487 Frankfurt am Main (DE)

(56) Entgegenhaltungen:
- HELVETICA CHIMICA ACTA, Bd. 24, Nr. 2, 15. M rz 1941, Basel, CH, Seiten 197 - 212 P. RUGGLI, ET AL.: 'Die S urechloride der m-Sulfo-benzoes ure und ihre Umstzungen mit Aminen und Phenolen'
- J. MARCH: 'ADVANCED ORGANIC CHEMISTRY, 3rd Edition,' 1985 , JOHN WILEY & SONS , NEW YORK, US

## Beschreibung

### Verfahren zur Herstellung von 3-Sufobenzoesäure und deren Alkalisalzen

Die vorliegende Erfindung betrifft ein sich gegenüber dem Stand der Technik durch einen verringerten Anfall von Abwasser auszeichnendes Verfahren zur Herstellung von 3-Sulfobenzoesäure und/oder deren Alkalisalzen.

Es ist aus Offermann, Liebigs Annalen der Chemie 280, 6 (1894) bekannt, das Natriumsalz der 3-Sulfobenzoesäure durch Sulfonierung von Benzoesäure mit Oleum bei 200°C, Eintragen des resultierenden Reaktionsproduktes in ein Wasser-Eis-Gemisch und Zusatz von Natriumchlorid herzustellen. Nachteilig an diesem Verfahren ist, daß man eine Natriumchlorid enthaltende, verdünnte Schwefelsäure erhält, die technisch nur unter großem Aufwand aufgearbeitet werden kann. Aufgrund des hohen Natriumchlorid-Gehaltes enthält das Reaktionsprodukt lediglich 75 % des Natriumsalzes der 3-Sulfobenzoesäure.

Nach dem in der US 4 358 410 beschriebenen Verfahren wird Benzoesäure mit Oleum bei 130°C umgesetzt und das anfallende Reaktionsgemisch anschließend mit einer wäßrigen, konzentrierten Natriumchloridlösung versetzt und abgekühlt. Dies führt zwar zu einer verbesserten Reinheit des Produktes, aber auch zu einem großen Anteil schwer zu entsorgender, verdünnter Schwefelsäure, die mit Natriumchlorid verunreinigt ist.

Nach einem von Ruggli und Grün in Helv. Chim. Acta 24, 197 (1941) beschriebenen Verfahren erhält man freie 3-Sulfobenzoesäure durch Umsetzung von 3-Benzoesäuresulfochlorid mit der 10-fachen Menge Wasser unter Erwärmen. Anschließend wird das Wasser abdestilliert, wobei ein Sirup erhalten wird. Man versetzt diesen Sirup mit frischem Wasser und destilliert das Wasser anschließend wieder ab. Dieser Vorgang wird zweimal wiederholt. Der Nachteil dieses Verfahrens besteht darin, daß dreimal große Mengen Wasser, die nicht wieder verwendet werden können, abdestilliert werden müssen. Dies erfordert einen sehr hohen Energiebedarf. Zudem ist eine technische Realisierung nur unter Schwierigkeiten möglich, da im Reaktionsgefäß 3-Sulfobenzoesäure zurückbleibt, die als Festprodukt bei 150°C schmilzt und demzufolge nur als heiße Schmelze aus dem Reaktionsgefäß abgelassen werden kann. Die DE 3 122 264 und Ind. Eng. Chem. 45, 2065 (1953) betreffen ein Verfahren zur Herstellung von 3-Sulfobenzoesäure durch Umsetzung von Benzoesäure mit einer stöchiometrischen Menge Schwefeltrioxid. Hierbei verbleibt jedoch ebenfalls ein Festprodukt im Reaktor, das nur in schmelzflüssiger Form abgelassen werden kann. Dies zieht einen erhöhten technischen Aufwand nach sich und ergibt Probleme bei der Weiterverarbeitung. Zudem ist die Handhabung von Schwefeltrioxid nicht unproblematisch und erfordert besondere Vorkehrungen.

Es besteht daher ein Bedarf nach einem Verfahren, das sich nicht nur technisch leicht durchführen läßt, sondern auch zu einer Verringerung der Abwassermenge führt. Es soll darüber hinaus sicherstellen, daß das gewünschte Wertprodukt sowohl in hoher Ausbeute als auch in großer Reinheit anfällt.

Gelöst wird diese Aufgabe durch ein Verfahren zur Herstellung von 3-Sulfobenzoesäure und/oder deren Alkalisalzen. Es ist dadurch gekennzeichnet, daß man 3-Benzoesäuresulfochlorid mit Wasser und einem mit Wasser nicht mischbaren, 3-Sulfobenzoesäure nicht oder nur gering lösendem Lösungsmittel vermischt, aus dem Gemisch Wasser durch Azeotropdestillation entfernt, abkühlt und die gebildete 3-Sulfobenzoesäure gegebenenfalls mit Alkalihydroxid und/oder einer Alkalihydroxid bildenden Substanz versetzt und Wasser erneut durch Azeotropdestillation entfernt.

Das Einsatzmaterial 3-Benzoesäuresulfochlorid läßt sich nach dem beispielsweise in der DE 3 919 840 beschriebenen Verfahren herstellen. Es kann vorteilhafterweise in wasserfeuchtem Zustand eingesetzt werden. Sein Wassergehalt beträgt üblicherweise 20 bis 40 Gew.-%.

Das erfindungsgemäße Verfahren läßt sich sowohl mit vergleichsweise recht geringen als auch mit relativ höheren Wassermengen durchführen. Üblicherweise setzt man 0,1 bis 10, insbesondere 0,3 bis 4, bevorzugt 0,4 bis 2 Gew.-Teile Wasser je Gew.-Teil 3-Benzoesäuresulfochlorid ein.

Ein weiteres Merkmal des Verfahrens besteht darin, ein mit Wasser nicht mischbares Lösungsmittel, das die 3-Sulfobenzoesäure nicht oder nur in geringem Umfang löst, einzusetzen. Als zweckmäßig hat es sich erwiesen, hierfür einen aromatischen Kohlenwasserstoff zu verwenden.

Geeignete Lösungsmittel sind beispielsweise Toluol, Mesitylen, o-Xylol, m-Xylol, p-Xylol, Gemische verschiedener Xylole, Chlorbenzol und/oder Dichlorbenzol. Es lassen sich auch Mischungen der vorstehend genannten Solventien einsetzen. Mit gutem Erfolg lassen sich Toluol und/oder Mesitylen, insbesondere Toluol als Lösungsmittel gebrauchen.

Ebenfalls gut geeignet sind o-Xylol, m-Xylol,und/oder p-Xylol sowie deren Gemische, insbesondere wie sie als technisches Produkt anfallen.

Ferner haben sich chlorierte aromatische Kohlenwasserstoffe, beispielsweise Chlorbenzol und/oder Dichlorbenzol, insbesondere Chlorbenzol als brauchbare Lösungsmittel erwiesen. Es läßt sich jedoch jedes Lösungsmittel, das sich mit Wasser nicht mischt und als azeotropes Wasserschleppmittel dient, verwenden, falls es zusätzlich das Wertprodukt, nämlich die 3-Sulfobenzoesäure nicht oder nur in geringen Umfange löst. Die vorstehend genannten Lösungsmittel stellen keine vollständige Aufzählung, sondern lediglich eine Auswahl brauchbarer Solventien dar.

Das Verfahren läßt sich unter Zusatz vergleichweise niedriger Lösungsmittelmengen, aber auch in Gegenwart relativ großer Lösungsmittelmengen mit gutem Erfolg praktizieren. Üblicherweise setzt man 0,8 bis 10, insbesondere 1,5 bis 3 Gew.-Teile Lösungsmittel je Gew.-Teil 3-Benzoesäuresulfochlorid ein. Es ist allerdings auch möglich mit niedrigeren oder höheren Mengen an Solvens, als vorstehend angegeben, zu arbeiten.

Zur Durchführung des Verfahrens versetzt man das 3-Benzoesäuresulfochlorid mit Wasser und dem Lösungsmittel und sorgt für eine intensive Durchmischung. Anschließend erhitzt man das resultierende Gemisch bis zum Sieden und kreist Wasser mittels Azeotropdestillation aus. Das dabei im Destillat anfallende Lösungsmittel wird abgetrennt und wieder in die Azeotropdestillation eingesetzt.

Die azeotrope Wasserabtrennung kann man wahlweise bei reduziertem Druck, bei Atmosphärendruck oder bei erhöhtem Druck ausführen. Besonders einfach gestaltet sich die Wasserabtrennung bei Atmosphärendruck. Deshalb wird man dieser Variante häufig den Vorzug geben.

Nach dem das Wasser restlos aus dem Reaktionsgemisch entfernt ist, also kein Wasser mehr als Azeotrop anfällt, kühlt man die aus Lösungsmittel und auskristallisierter 3-Sulfobenzoesäure bestehende Suspension ab. Üblicherweise genügt es, eine Temperatur von 40 bis 5°C, insbesondere Raumtemperatur, einzustellen. Anschließend trennt man die auskristallisierte 3-Sulfobenzoesäure, beispielsweise durch Filtration oder Zentrifugieren, ab und trocknet sie erforderlichenfalls.

Das Verfahren zeichnet sich nicht nur durch eine hohe Ausbeute, sondern auch durch eine besondere Reinheit der 3-Sulfobenzoesäure aus. Insbesondere enthält die derart hergestellte Sulfobenzoesäure kein Chlorid, beispielsweise in Form von Alkalichloriden. Die Ausbeute beträgt > 94 % der Theorie. Die 3-Sulfobenzoesäure weist eine Reinheit von > 97 % auf.

Ein besonderer Vorteil des Verfahrens besteht darin, daß das mittels Azeotropdestillation abgetrennte Wasser erneut für die Hydrolyse von 3-Benzoesäuresulfochlorid verwendet werden kann. Dies führt zu einer weiteren Verringerung von bei der Umsetzung anfallendem Abwasser. Zudem ist es nur in geringem Maße mit Schadstoffen, insbesondere HCl belastet. Der übliche Anfall großer Mengen Dünnsäure (wäßrige Natriumchlorid enthaltende Schwefelsäure) wird vermieden und dadurch die durch Abwässer verursachte Umweltbelastung erheblich reduziert.

Ferner ist es überraschend, daß das erfindungsgemäße Verfahren trotz des Auskreisens von Wasser bei relativ hohen Temperaturen nicht zur Abspaltung von Wasser aus der gebildeten 3-Sulfobenzoesäure und somit zum Entstehen entsprechender Säureanhydride führt.

Will man die Alkalisalze der 3-Sulfobenzoesäure herstellen, so geht man entweder von erfindungsgemäß hergestellter, bereits abgetrennter, getrockneter 3-Sulfobenzoesäure aus oder verwendet vorteilhaft das bei der vorstehend beschriebenen Herstellung anfallende Gemisch, das nach der Wasserabtrennung als eine Suspension von 3-Sulfobenzoesäure im Lösungsmittel vorliegt. Man kann diese Suspension unmittelbar nach Absenken der Temperatur, beispielsweise auf etwa 100°C oder darunter, mit Alkalihydroxid oder einer Alkalihydroxid bildenden Substanz, insbesondere einer wäßrigen Lösung des Alkalihydroxids oder der Alkalihydroxid bildenden Substanz, versetzen und das Wasser wiederum mit Hilfe des Lösungsmittels azeotrop abdestillieren. Als Alkalihydroxid können Natriumhydroxid oder Kaliumhydroxid verwendet werden. Als Alkalihydroxid bildende Substanzen kommen beispielsweise Alkalioxide, Alkalicarbonate oder Alkalihydrogencarbonate, insbesondere Oxide, Carbonate oder Hydrogencarbonate des Natriums oder Kaliums in Betracht.

Besonders einfach läßt sich das erfindungsgemäße Verfahren mittels wäßriger Alkalihydroxidlösungen ausüben. Üblicherweise gebraucht man wäßrige Lösungen mit 5 bis 50, insbesondere 20 bis 40 Gew.-% Alkalihydroxid.

Das Gemisch wird erhitzt und das Wasser als Azeotrop abgetrennt. Das destillativ abgetrennte Wasser läßt sich ohne zusätzliche Behandlung wieder in das Verfahren einsetzen. Es läßt sich entweder ganz oder zum Teil in der Stufe der Hydrolyse verwenden oder kann wiederum zur Herstellung der wäßrigen, Alkalihydroxid oder eine Alkalihydroxid bildende Substanz enthaltenden Lösung gebraucht werden. Auf diese Weise wird wiederum die gesamte Menge Abwasser je Ansatz verringert.

Nach destillativer Abtrennung des Wassers kühlt man die aus Alkalisalzen der 3-Sulfobenzoesäure und Lösungsmittel bestehende Suspension, üblicherweise auf 40 bis 5°C, insbesondere Raumtemperatur herunter, trennt die Alkalisalze beispielsweise mittels Filtration oder Zentrifugieren ab und entfernt restliches Lösungsmittel gegebenenfalls durch Waschen beispielsweise mit Aceton, Methylethylketon, n-Propanol und/oder i-Propanol und nachfolgender Trocknung.

Erfindungsgemäß erhält man Alkalisalze der 3-Sulfobenzoesäure, die frei von Alkalichloriden sind. Die Ausbeute liegt oberhalb von 93 %. Die Reinheit des Wertprodukts beträgt mindestens 97 %.

Die nachfolgenden Beispiele belegen die Erfindung, ohne sie zu beschränken.

### Experimenteller Teil

### Beispiel 1

### Herstellung von 3-Sulfobenzoesäure

300 g 3-Benzoesäuresulfochlorid mit einem Wassergehalt von 38,8 %, (entsprechend 183,6 g (0,83 mol) Benzoesäuresulfochlorid 100 %ig) werden mit 150 g Wasser versetzt und 1 Stunde auf 100°C erhitzt. Dann werden innerhalb von 1 Stunde 600 g Xylol zugetropft und insgesamt 260 ml Wasser azeotrop mit Xylol abdestilliert. Die Reaktionsmischung wird abgekühlt und das ausgefallene Produkt abgesaugt. Nach Trocknen bei 100°C/100 Torr (13,16 kPa) erhält man 164,7 g 3-Sulfobenzoesäure, die nach Bestimmung mittels Titration eine Reinheit von 97,7 % aufweist; dies entspricht einer Ausbeute von 95,6 %.

### Beispiel 2

### Herstellung von 3-Sulfobenzoesäure

300 g 3-Benzoesäuresulfochlorid mit einem Wassergehalt von 38,8 %, (entsprechend 183,6 g (0,83 mol) Benzoesäuresulfochlorid 100 %ig) werden mit 100 g Wasser versetzt und 1 Stunde auf 100°C erhitzt. Dann werden innerhalb von 1 Stunde 500 g Toluol zugetropft und insgesamt 164 ml Wasser azeotrop mit Toluol abdestilliert. Die Reaktionsmischung wird abgekühlt und das ausgefallene Produkt abgesaugt. Nach Trocknen bei 100°C/100 Torr (13,16 kPa) erhält man 163,5 g 3-Sulfobenzoesäure, die nach Bestimmung mittels Titration eine Reinheit von 98,2 % aufweist; dies entspricht einer Ausbeute von 95,4 %.

### Beispiel 3

### Herstellung von 3-Sulfobenzoesäure

250 g 3-Benzoesäuresulfochlorid mit einem Wassergehalt von 35,9 %, (entsprechend 160,2 g (0,73 mol) Benzoesäuresulfochlorid 100 %ig) werden mit 86 g Wasser und 500 g Xylol versetzt und an einem Wasserabscheider erhitzt. Insgesamt werden 171 ml Wasser ausgekreist. Die Reaktionsmischung wird abgekühlt und das das ausgefallene Produkt abgesaugt. Nach Trocknen bei 100°C/100 Torr (13,16 kPa) erhält man 144,3 g 3-Sulfobenzoesäure, die nach Bestimmung mittels Titration eine Reinheit von 97,5 % aufweist; dies entspricht einer Ausbeute von 95,6 %.

### Beispiel 4

### Herstellung von 3-Sulfobenzoesäure Natriumsalz

565,0 g 3-Benzoesäuresulfochlorid mit einem Wassergehalt von 32,9 %, (entsprechend 379,1 g (1,72 mol) Benzoesäuresulfochlorid 100 %ig) werden mit 182 g Wasser und 1170 g Xylol versetzt und an einem Wasserabscheider erhitzt. Insgesamt werden 340 ml Wasser azeotrop mit Xylol abdestilliert. Nach Abkühlen der Reaktionsmischung auf 25°C tropft man innerhalb von 30 Minuten 210 g (1,73 mol) Natronlauge 33%ig zu. Anschließend werden erneut 170 ml Wasser ausgekreist. Nach Abkühlen wird das ausgefallene Produkt abgesaugt. Nach Trocknen bei 100°C/100 Torr (13,16 kPa) erhält man 370,2 g 3-Sulfobenzoesäure Natriumsalz, das nach Bestimmung mittels Titration eine Reinheit von 98,3 % aufweist; dies entspricht einer Ausbeute von 94,3 %.

### Beispiel 5

### Herstellung von 3-Sulfobenzoesäure Natriumsalz

273,6 g 3-Benzoesäuresulfochlorid mit einem Wassergehalt von 28,6 %, (entsprechend 195,3 g (0,89 mol) Benzoesäuresulfochlorid 100 %ig) werden mit 50 g Wasser und 500 g Chlorbenzol versetzt und an einem Wasserabscheider erhitzt. Insgesamt werden 106 ml Wasser ausgekreist. Nach Abkühlen der Reaktionsmischung auf 100°C tropft man innerhalb von 30 Minuten 108 g (0,89 mol) Natronlauge 33%ig zu. Anschließend werden erneut 90 ml Wasser ausgekreist. Nach Abkühlen wird das ausgefallene Produkt abgesaugt. Nach Trocknen bei 100°C/100 Torr (13,16 kPa) erhält man 190,4 g 3-Sulfobenzoesäure Natriumsalz, das nach Bestimmung mittels Titration eine Reinheit von 97,3 % aufweist; dies entspricht einer Ausbeute von 93,3 %.

### Vergleichsbeispiel 1

### Herstellung von 3-Sulfobenzoesäure-Natriumsalz gemäß US 4 358 410

In einem 1l-Vierhalskolben, bestückt mit Rührer, Innenthermometer und Rückflußkühler, werden 122,1 g (1,0 mol) Benzoesäure bei 125 bis 130°C geschmolzen. Dann werden innerhalb von 30 Minuten 405 g Oleum eingetragen. Anschließend erhitzt man das Reaktionsgemisch auf 130°C und rührt 1 Stunde bei dieser Temperatur nach. Das Reaktionsgemisch wird abgekühlt und innerhalb von 15 Minuten in 1800 g (= 1500 ml) Natriumchloridlösung 26,4 %ig einlaufen gelassen. Die Reaktionsmischung wird auf 80°C bis 85°C erwärmt. Danach läßt man das Gemisch unter Rühren auf 50°C abkühlen. Anschließend wird mit einem Eisbad auf < 5°C abgekühlt und noch 30 Minuten bei dieser Temperatur nachgerührt. Das ausgefallene Produkt wird abgesaugt und mit 256 (= 250 ml) 5°C kalter Natriumchloridlösung 5%ig, in 2 Portionen gewaschen. Man erhält nach Trocknen 204,4 g 3-Sulfobenzoesäure-Natriumsalz tel quel (Gehalt 96 %; Natriumchlorid-Gehalt: 3 %), dies entspricht 196,3 g (0,88 mol) 3-Sulfobenzoesäure-Natriumsalz 100 %ig gerechnet. Es fallen 2342 g Abwasser an.

### Vergleichsbeispiel 2

### Herstellung von 3-Benzoesäuresulfochlorid gemäß DE 39 19 840, Beispiel 16

In einem 2|-Vierhalskolben, bestückt mit Rührer, Innenthermometer, Rückflußkühler mit Gasableitung und Tropftrichter, werden 349,5 g = 199,7 ml (3,0 mol) Chlorsulfonsäure und 20,0 g Schwefelsäure 96 %ig bei 25°C vorgelegt und 1,0 g Amidosulfonsäure zugesetzt. Anschließend werden in diese Mischung 122,1 g (1,0 mol) Benzoesäure eingetragen. Die Reaktionsmischung wird innerhalb von 3 Stunden auf 120°C aufgeheizt und bis zum Ende der HCl-Entwicklung nachgerührt (ca. 30 Minuten). Danach wird auf 70°C abgekühlt und es werden innerhalb von 2 Stunden 119,0 g (1,0 mol) Thionylchlorid zugetropft. Die Reaktionslösung wird nun auf 80°C erwärmt und ca. 30 Minuten bis zum Ende der Gasentwicklung nachgerührt. Das Gemisch wird in 1000 g Eiswasser bei 10°C innerhalb von 2 Stunden getropft. Das ausgefallene Produkt wird abgesaugt und mit 500 g Eiswasser gewaschen. Man erhält 321,2 g 3-Benzoesäuresulfochlorid tel quel (Wassergehalt: 33,9 %; Chlorwert: 10,8 %), entsprechend 212,3 g (0.96 mol) 3-Benzoesäuresulfochlorid 100 %ig. Es fallen 1115 g Mutterlauge und 538 g Waschwasser an.

Wie die in der nachfolgenden Tabelle zusammengefaßte Gegenüberstellung zeigt, führt die Kombination des in der DE 39 19 840 beschriebenen Verfahrens mit dem erfindungsgemäßen Verfahren im Vergleich zum Stand der Technik, repräsentiert durch die US 4 358 410, zu einer Verringerung nicht nur der Abwassermenge sondern auch zu einer Abnahme der im Abwasser enthaltenden Abfallstoffe.

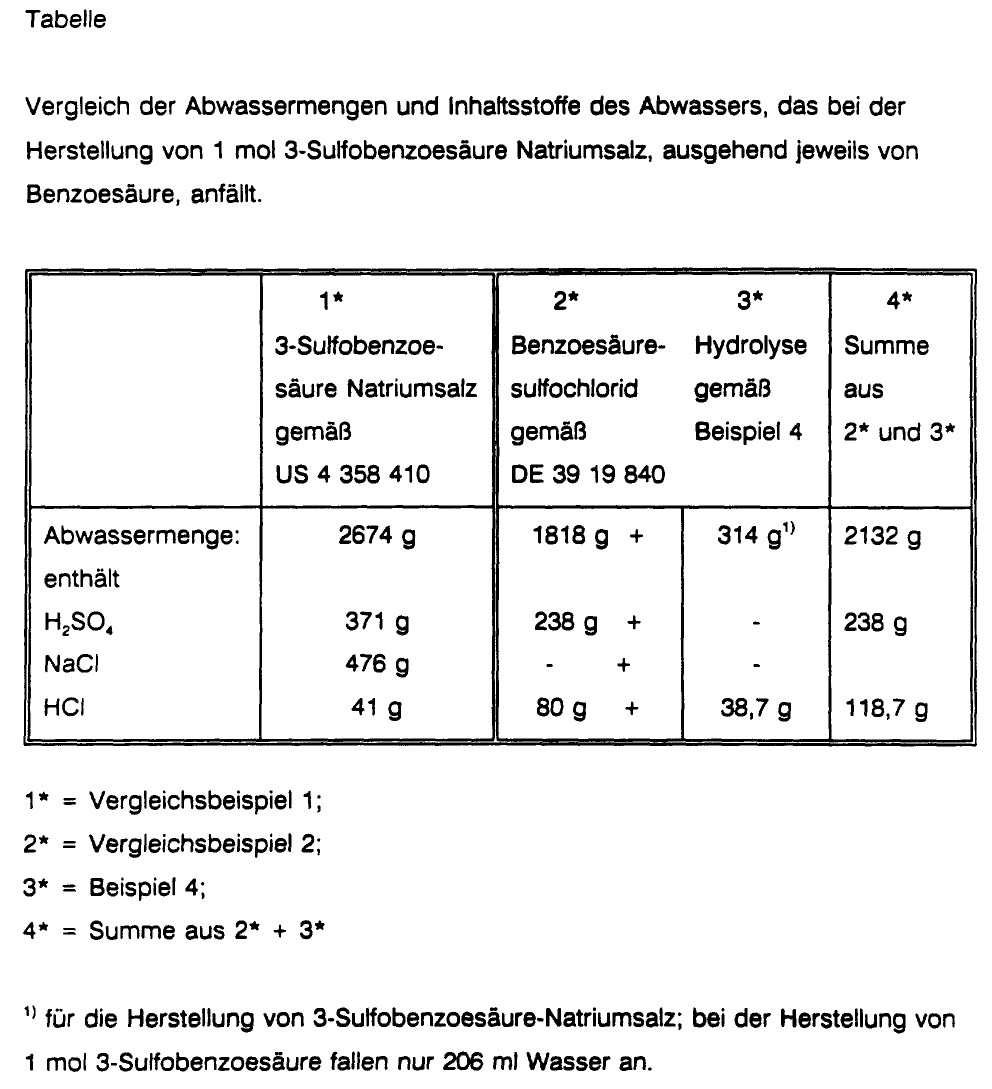

## Patentansprüche

1. Verfahren zur Herstellung von 3-Sulfobenzoesäure und/oder deren Alkalisalzen, dadurch gekennzeichnet, daß man 3-Benzoesäuresulfochlorid mit Wasser und einem mit Wasser nicht mischbaren, 3-Sulfobenzoesäure nicht oder nur gering lösendem Lösungsmittel vermischt, aus dem Gemisch Wasser durch Azeotropdestillation entfernt, abkühlt und die gebildete 3-Sulfobenzoesäure gegebenenfalls mit Alkalihydroxid und/oder einer Alkalihydroxid bildenden Substanz versetzt und Wasser erneut durch Azeotropdestillation entfernt.

2. Verfahren nach Anspruch 1, dadurch gekennzeichnet, daß man 0,1 bis 10, insbesondere 0,3 bis 4, bevorzugt 0,4 bis 2 Gew.-Teile Wasser je Gew.-Teil 3-Benzoesäuresulfochlorid einsetzt.

3. Verfahren nach Anspruch 1 oder 2, dadurch gekennzeichnet, daß man als mit Wasser nicht mischbares, 3-Sulfobenzoesäure nicht oder nur gering lösendes Lösungsmittel einen aromatischen Kohlenwasserstoff einsetzt.

4. Verfahren nach einem oder mehreren der Ansprüche 1 bis 3, dadurch gekennzeichnet, daß man als Lösungsmittel Toluol, Mesitylen, o-Xylol, m-Xylol, p-Xylol, Chlorbenzol und/oder Dichlorbenzol einsetzt.

5. Verfahren nach einem oder mehreren der Ansprüche 1 bis 4, dadurch gekennzeichnet, daß man als Lösungsmittel Toluol und/oder Mesitylen, insbesondere Toluol einsetzt.

6. Verfahren nach einem oder mehreren der Ansprüche 1 bis 4, dadurch gekennzeichnet, daß man als Lösungsmittel o-Xylol, m-Xylol und/oder p-Xylol einsetzt.

7. Verfahren nach einem oder mehreren der Ansprüche 1 bis 4, dadurch gekennzeichnet, daß man als Lösungsmittel Chlorbenzol und/oder Dichlorbenzol, insbesondere Chlorbenzol einsetzt.

8. Verfahren nach einem oder mehreren der Ansprüche 1 bis 7, dadurch gekennzeichnet, daß man 0,8 bis 10, insbesondere 1,5 bis 3 Gew.-Teile Lösungsmittel je Gew.-Teil 3-Benzoesäuresulfochlorid einsetzt.

9. Verfahren nach einem oder mehreren der Ansprüche 1 bis 8, dadurch gekennzeichnet, daß man die gebildete 3-Sulfobenzoesäure mit der äquivalenten Menge Alkalihydroxid, insbesondere einer wäßrigen Alkalihydroxid-Lösung versetzt.

10. Verfahren nach einem oder mehreren der Ansprüche 1 bis 8, dadurch gekennzeichnet, daß man die gebildete 3-Sulfobenzoesäure mit der äquivalenten Menge einer Alkalihydroxid bildenden Substanz, insbesondere einer wäßrigen Alkalihydrogencarbonat- und/oder Alkalicarbonat-Lösung versetzt.

## Claims

1. A process for preparing 3-sulfobenzoic acid and/or alkali metal salts thereof, which comprises mixing 3-(chlorosulfonyl)benzoic acid with water and a water-immiscible solvent in which 3-sulfobenzoic acid is insoluble or only sparingly soluble, removing water from the mixture by azeotropic distillation, cooling and, if desired, admixing the 3-sulfobenzoic acid formed with alkali metal hydroxide and/or a substance forming alkali metal hydroxide and again removing water by azeotropic distillation.

2. The process as claimed in claim 1, wherein from 0.1 to 10, in particular from 0.3 to 4, preferably from 0.4 to 2 parts by weight of water are used per part by weight of 3-(chlorosulfonyl)benzoic acid.

3. The process as claimed in claim 1 or 2, wherein the water-immiscible solvent used in which 3-sulfobenzoic acid is insoluble or only sparingly soluble is an aromatic hydrocarbon.

4. The process as claimed in one or more of claims 1 to 3, wherein the solvent used is toluene, mesitylene, o-xylene, m-xylene, p-xylene, chlorobenzene and/or dichlorobenzene.

5. The process as claimed in one or more of claims 1 to 4, wherein the solvent used is toluene and/or mesitylene, in particular toluene.

6. The process as claimed in one or more of claims 1 to 4, wherein the solvent used is o-xylene, m-xylene and/or p-xylene.

7. The process as claimed in one or more of claims 1 to 4, wherein the solvent used is chlorobenzene and/or dichlorobenzene, in particular chlorobenzene.

8. The process as claimed in one or more of claims 1 to 7, wherein from 0.8 to 10, in particular from 1.5 to 3 parts by weight of solvent are used per part by weight of 3-(chlorosulfonyl)benzoic acid.

9. The process as claimed in one or more of claims 1 to 8, wherein the 3-sulfobenzoic acid formed is admixed with the equivalent amount of alkali metal hydroxide, in particular an aqueous alkali metal hydroxide solution.

10. The process as claimed in one or more of claims 1 to 8, wherein the 3-sulfobenzoic acid formed is admixed with the equivalent amount of a substance forming alkali metal hydroxide, in particular an aqueous alkali metal hydrogen carbonate and/or alkali metal carbonate solution.

## Revendications

1. Procédé de préparation d'acide 3-sulfobenzoïque et/ou de ses sels de métaux alcalins, caractérisé en ce que l'on mélange de l'acide 3-chlorosulfonylbenzoïque avec de l'eau et un solvant non miscible à l'eau dans lequel l'acide 3-sulfobenzoïque est insoluble ou n'est que peu soluble, on sépare l'eau du mélange par distillation azéotropique, on refroidit et, éventuellement, on ajoute à l'acide 3-sulfobenzoïque formé un hydroxyde de métal alcalin et/ou une substance formant un hydroxyde de métal alcalin et on sépare de nouveau l'eau par distillation azéotropique.

2. Procédé selon la revendication 1, caractérisé en ce que l'on utilise 0,1 à 10, en particulier 0,3 à 4, de préférence 0,4 à 2 parties en masse d'eau par partie en masse d'acide 3-chlorosulfonylbenzoïque.

3. Procédé selon la revendication 1 ou 2, caractérisé en ce que l'on utilise un hydrocarbure aromatique comme solvant non miscible à l'eau dans lequel l'acide 3-sulfobenzoïque est insoluble ou n'est que peu soluble.

4. Procédé selon l'une ou plusieurs des revendications 1 à 3, caractérisé en ce que l'on utilise comme solvant du toluène, du mésitylène, de l'o-xylène, du m-xylène, du p-xylène, du chlorobenzène et/ou un dichlorobenzène.

5. Procédé selon l'une ou plusieurs des revendications 1 à 4, caractérisé en ce que l'on utilise comme solvant du toluène et/ou du mésitylène, en particulier du toluène.

6. Procédé selon l'une ou plusieurs des revendications 1 à 4, caractérisé en ce que l'on utilise comme solvant de l'o-xylène, du m-xylène et/ou du p-xylène;

7. Procédé selon l'une ou plusieurs des revendications 1 à 4, caractérisé en ce que l'on utilise comme solvant du chlorobenzène et/ou un dichlorobenzène, en particulier du chlorobenzène.

8. Procédé selon l'une ou plusieurs des revendications 1 à 7, caractérisé en ce que l'on utilise 0,8 à 10, en particulier 1,5 à 3 parties en masse de solvant par partie en masse d'acide 3-chlorosulfonylbenzoïque.

9. Procédé selon l'une ou plusieurs des revendications 1 à 8, caractérisé en ce que l'on ajoute à l'acide 3-sulfobenzoïque formé une quantité équivalente d'hydroxyde de métal alcalin, en particulier d'une solution aqueuse d'hydroxyde de métal alcalin.

10. Procédé selon l'une ou plusieurs des revendications 1 à 8, caractérisé en ce que l'on ajoute à l'acide 3-sulfobenzoïque formé une quantité équivalente d'une substance formant un hydroxyde de métal alcalin, en particulier d'une solution aqueuse d'un hydrogénocarbonate de métal alcalin et/ou de carbonate de métal alcalin.
